(19) Europäisches Patentamt
European Patent Office
Office européen des brevets

(11) EP 2 107 417 A1

(12) EUROPEAN PATENT APPLICATION

(43) Date of publication:
07.10.2009 Bulletin 2009/41

(51) Int Cl.:
G02C 7/04 (2006.01)    A61F 2/16 (2006.01)

(21) Application number: 09156602.6

(22) Date of filing: 30.03.2009

(84) Designated Contracting States:
AT BE BG CH CY CZ DE DK EE ES FI FR GB GR
HR HU IE IS IT LI LT LU LV MC MK MT NL NO PL
PT RO SE SI SK TR
Designated Extension States:
AL BA RS

(30) Priority: 31.03.2008 JP 2008091764

(71) Applicant: Nidek Co., Ltd.
Gamagori-shi
Aichi 443-0038 (JP)

(72) Inventors:
• Isogai, Aki
  Gamagori-shi, Aichi 443-0038 (JP)
• Taki, Seiji
  Gamagori-shi, Aichi 443-0038 (JP)
• Nakahata, Yoshihiro
  Gamagori-shi, Aichi 443-0038 (JP)
• Sunada, Tsutomu
  Gamagori-shi, Aichi 443-0038 (JP)

(74) Representative: Prüfer & Partner GbR
European Patent Attorneys
Sohnckestraße 12
81479 München (DE)

(54) **Design method of aspheric ophthalmic lens**

(57)    A method of designing an aspheric ophthalmic lens capable of reducing aberration of an eye, comprises: a step of modeling an optical system including a cornea and an ophthalmic lens by inclining the optical system about a rotation point by a predetermined angle in consideration of a displacement angle between an optical axis and a visual axis of the eye (1); a step of calculating aberration resulting from the modeled optical system; and a step of designing a shape of at least one of a front surface and a back surface of the ophthalmic lens to reduce the aberration resulting from the modeled optical system.

## FIG. 1

**Description**

Technical Field

The present invention relates to a design method of an ophthalmic lens to be worn on or set in an eye in use, such as an intraocular lens and a contact lens.

Background Art

Heretofore, there have been known ophthalmic lenses such as an intraocular lens to be set in an eye in place of a crystalline lens and a contact lens to be placed on a cornea. Those ophthalmic lenses mostly have spherical surfaces. However, there has recently been proposed an ophthalmic lens having an aspheric surface optically designed to reduce the effect of aberration of an eye to obtain shaper images.

Such aspheric ophthalmic lens is designed to reduce the effect of eye aberration as mentioned above. This lens is produced by optical design with reference to an optical axis of the eye, but without consideration of a visual axis of the eye.

Summary of Invention

Technical Problem

The present invention has a purpose to provide a design method of aspheric ophthalmic lens capable of reducing the effect of aberration of an eye by taking into account a visual axis of the eye.

Solution to Problem

To achieve the above purpose, the present invention provides a method of designing an aspheric ophthalmic lens capable of reducing aberration of an eye, comprising: a step of modeling an optical system including a cornea and an ophthalmic lens by inclining the optical system about a rotation point by a predetermined angle in consideration of a displacement angle between an optical axis and a visual axis of the eye; a step of calculating aberration resulting from the modeled optical system; and a step of designing a shape of at least one of a front surface and a back surface of the ophthalmic lens to reduce the aberration resulting from the modeled optical system.

Further developments of the present invention are given in the dependent claims.

Brief Description of Drawings

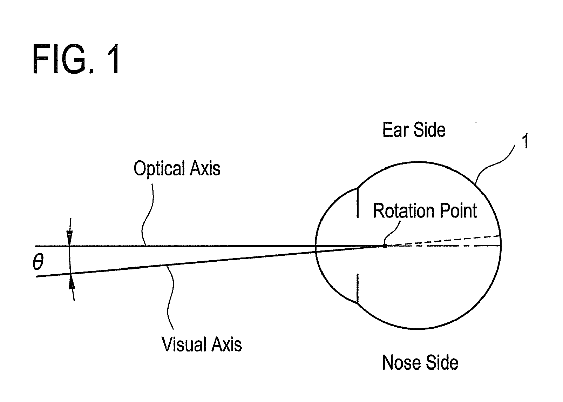

Fig. 1 is a schematic view showing displacement between an optical axis and a visual axis of an eye.

Description of Embodiments

A detailed description of a preferred embodiment of the present invention will now be given referring to the accompanying drawing. In this embodiment, an ophthalmic lens is exemplified as an intraocular lens ("IOL") which serves as an alternative to a crystalline lens of an eye. In this embodiment, the IOL is optically designed in a way of setting a model by inclining an optical system including a cornea and the IOL at a predetermined angle in consideration of displacement between an optical axis and a visual axis of an eye, and designing the IOL by use of this model to reduce the effect of aberration of the eye.

Fig. 1 is a schematic view showing displacement between an optical axis and a visual axis of an eye 1 viewed from above. A central fovea is slightly deviated to an ear side than the optical axis of the eye 1. It is therefore well known that the visual axis is not coaxial with the optical axis of the eye and is inclined at a predetermined angle with respect to the optical axis of the eye. However, it is difficult to exactly determine the visual axis. In this embodiment, accordingly, it is assumed that a point on the optical axis located closer to a fundus from a corneal vertex by a predetermined distance (e.g., 7.2 mm) is a rotation point of the eye and an axis horizontally inclined by a predetermined angle θ relative to the optical axis is treated as the visual axis. A displacement angle θ between the optical axis and the visual axis in this embodiment is set at 5˚ but it is not limited thereto. This displacement angle θ may be set in a range of 1˚ to 10˚ and more preferably 3˚ to 7˚ based on for example an empirical rule.

The central fovea is slightly deviated to the ear side relative to the optical axis as mentioned above. More specifically, the central fovea is displaced to the ear side relative to the optical axis and also located slightly above a horizontal plane including the optical axis. Thus, the visual axis also has a slight displacement angle with respect to the optical axis in a vertical direction. When the IOL is to be optically designed by taking into account the displacement angle in the vertical direction, the displacement angle can be set to be preferably 3° or less, more preferably in a range of 1° to 2° with respect to the optical axis by centering the rotation point, in addition to the aforementioned <u>horizontal</u> displacement angle.

For performing optical design of the IOL, an existing optical simulation software (e.g., ZEMAX) is used. The optical design in this embodiment is conducted by modeling the optical system including the cornea and the IOL. Modeling such optical system requires a corneal aspheric polynomial coefficient, a curvature (a radius of curvature) of a cornea, a refractive index of the cornea, a distance from the cornea to the IOL, and power or diopter (refractive power) of the IOL. As design information to determine the power of the IOL, a curvature (a radius of curvature) of each of front and back lens surfaces, a refractive index of the IOL, and central thickness of the IOL are required. As information on a corneal shape, an average of quantitatively obtained data on human cornea may be used or information adopted in an existing model eye may be used. The corneal curvature may further be divided into a curvature of the corneal front surface and a curvature of the corneal back surface. In this embodiment, the aforementioned information for optical design is input by use of the optical simulation software, and the optical system including the cornea and the IOL is modeled by being inclined to a nose side by a predetermined angle (5°) about the rotation point and to a chin side by a predetermined angle (1°) about the rotation point. In this way, the axis relatively decentered by the predetermined angle about the rotation point with respect to the optical axis of the optical system including the cornea and the IOL is assumed to be the visual axis. Then, the IOL is optically designed into an aspheric shape to minimize aberration generated in the optical system with reference to the axis assumed to be the visual axis.

To determine aberration (wave aberration) generated in the modeled optical system, a Zernike polynomial is generally used. The Zernike polynomial can be described by the following mathematic expression (2).

$$\mathrm{w}(\rho, \ \theta)=\sum_{i=1}^{15} a_i Z_i \qquad \cdots (2)$$

Where, $Z_i$ is an i-th Zernike term, and $a_i$ is a weighting factor in this term. Table 1 shows 1st to 15th Zernike terms and aberrations, which are defined by the following expressions respectively.

Table 1

| i | $w_i(\rho, \theta)$ | |
|---|---|---|
| 1 | 1 | Piston |
| 2 | $2\rho\cos\theta$ | Inclination x |
| 3 | $2\rho\sin\theta$ | Inclination y |
| 4 | $\sqrt{3}(2\rho^2-1)$ | Defocus |
| 5 | $\sqrt{6}(\rho^2\sin 2\theta)$ | Primary Astigmatic Aberration |
| 6 | $\sqrt{6}(\rho^2\cos 2\theta)$ | Primary Astigmatic Aberration |
| 7 | $\sqrt{8}(3\rho^3-2\rho)\sin\theta$ | Coma Aberration |
| 8 | $\sqrt{8}(3\rho^3-2\rho)\cos\theta$ | Coma Aberration |
| 9 | $\sqrt{8}(3\rho^3\sin 3\theta)$ | 3 Foil |
| 10 | $\sqrt{8}(3\rho^3\cos 3\theta)$ | 3 Foil |

(continued)

| i | $w_i(\rho, \theta)$ | |
|---|---|---|
| 11 | $\sqrt{5}(6\rho^4 - 3\rho^2 + 1)$ | Spherical Aberration |
| 12 | $\sqrt{10}(4\rho^4 - 3\rho^2)\sin 2\theta$ | Secondary Astigmatic Aberration |
| 13 | $\sqrt{10}(4\rho^4 - 3\rho^2)\cos 2\theta$ | Secondary Astigmatic Aberration |
| 14 | $\sqrt{10}(4\rho^4\cos 4\theta)$ | 4 Foil |
| 15 | $\sqrt{10}(4\rho^4\sin 4\theta)$ | 4 Foil |

An IOL having a general spherical shape uses the 4th term. Furthermore, the aberration in the modeled optical system including the cornea and the IOL is usually reduced by bringing (approximating) a value obtained by use of the 11th term close to zero. In this embodiment, to determine the aberration generated in the state where the optical system is inclined about the rotation point by the predetermined angle, the coefficient $a_i$ is a value of the weighting coefficient based on the inclination by the predetermined angle.

In order to reduce the aberration in the thus modeled optical system, at least one of the front surface and the back surface of the IOL is designed as an aspheric shape. Each curved shape of the front surface and the back surface of the IOL can be described by the following mathematic expression (1).

$$z = \frac{(1/R)\ h^2}{1 + \sqrt{1 - (\frac{1}{R})^2(Q+1)\ h^2}} + A\,h^4 + B\,h^6 + C\,h^8 + D\,h^{10} \qquad \cdots(1)$$

Where "z" is a sag amount, "R" is a radius of curvature, "h" is a distance from a lens center, "Q" is a conic constant, and "A" to "D" are aspheric polynomial terms. In the case where the lens surface is treated as a spherical surface, not an aspheric surface, the conic constant and the aspheric polynomial terms are set to zero.

The values of the coefficients A to D used in the mathematic expression (1) are appropriately changed to bring (approximate) the value of the 11th term representing the spherical aberration in the Zernike polynomial to as close to zero as possible in the optical system modeled by use of the optical simulation software. This is repeated to determine the curved shape of the front and/or back surface of the IOL. It is also possible to determine the coefficients A to D by using a similar method taking into consideration the terms representing other aberrations such as the astigmatic aberration and the coma aberration as well as the term representing the spherical aberration in the Zernike polynomial.

The above embodiment is explained about the design method of the IOL to be used in place of the crystalline lens. The present invention is not limited thereto and may be applied to an ophthalmic lens such as a contact lens to be used for eyes.

<Example 1>

Example 1 for performing the IOL optical design using the aforementioned design method is explained below. Herein, the IOL of a refractive power of 20.0D is designed to design the IOL front surface into an aspheric shape. The optical simulation software used herein was ZEMAX. Various data needed for the design were set as follows:

| | |
|---|---|
| Corneal radius of curvature: | 7.575 mm, |
| Corneal refractive index: | 1.3375, |
| Corneal conic constant (Q): | -0.14135, |
| Distance from cornea to iris (pupil): | 3.6 mm, |
| Distance from iris to IOL front surface: | 0.9 mm, |

(continued)

| Radius of curvature of IOL front surface: | 13.36 mm, |
| Radius of curvature of IOL back surface: | -28.59 mm, |
| Refractive index of IOL base material: | 1.519, and |
| Design wavelength: | 587.56 nm. |

Based on the above data, the optical system including the cornea and the IOL obtained by use of the optical simulation software is inclined about the rotation point (7.2 mm from the corneal vertex) by 5° in the horizontal direction (to the nose side) and 1° in the vertical direction (to the chin side), thereby creating a starting model.

With such optical system model, the optical design was made by correcting the conic constant and 4th and 6th aspheric polynomial coefficients in the expression (1) with respect to the IOL front surface so that the 11th term in the above nominal becomes as close to zero as possible. This result is shown in Table 2. In Table 2, "Cornea" represents an aberration amount ($\mu$m) of only a set cornea, "Cornea + IOL" represents an aberration amount at the starting time, and "After Correction" represents an aberration amount indicated in the finally decided IOL optical design. Furthermore, the 4th and 6th aspheric polynomial coefficients finally decided are -5.518272E-04 and -2.919535E-05 respectively. The conic constant (Q) in the IOL front surface at that time was 0.767322 and the central thickness of the lens was 0.756 mm.

Table 2

| | Cornea | Cornea + IOL | After Correction |
|---|---|---|---|
| 1st | -0.595323468 | -0.743662252 | -0.067382968 |
| 2nd | 0.412809469 | -0.141036249 | 0.410480174 |
| 3rd | 0.082296591 | -0.028170886 | 0.081863017 |
| 4th | -0.002823135 | 0.001322866 | -0.008098441 |
| 5th | v0.01663683 | 0.039674746 | 0.023109376 |
| 6th | -0.040039552 | 0.095506008 | 0.055641174 |
| 7th | 0.030649127 | -0.010223299 | 0.029700738 |
| 8th | 0.153740727 | -0.051178866 | 0.148927832 |
| 9th | -0.000106208 | -0.0008264 | -0.000115897 |
| 10th | -0.000158442 | -0.001232564 | -0.000172908 |
| 11th | 0.272336146 | 0.344518689 | 0.026917963 |
| 12th | 8.34338E-07 | 0.002699888 | -0.000258909 |
| 13th | 1.4689E-07 | 0.001121567 | -0.000107671 |
| 14th | -3.81915E-07 | 8.30225E-06 | -4.45372E-06 |
| 15th | -3.64288E-07 | 8.361E-06 | -4.47135E-06 |

As shown in Table 2, in the modeled state where the optical system including the cornea and ophthalmic lens is inclined about the rotation point by the predetermined angle in consideration of the displacement angle between the optical axis and the visual axis of the eye, the IOL optical design could be made so as to minimize the aberration of the cornea.

While the presently preferred embodiment of the present invention has been shown and described, it is to be understood that this disclosure is for the purpose of illustration and that various changes and modifications may be made without departing from the scope of the invention as set forth in the appended claims.

## Claims

1. A method of designing an aspheric ophthalmic lens capable of reducing aberration of an eye, comprising:

a step of modeling an optical system including a cornea and an ophthalmic lens by inclining the optical system about a rotation point by a predetermined angle in consideration of a displacement angle between an optical axis and a visual axis of the eye (1);

a step of calculating aberration resulting from the modeled optical system; and

a step of designing a shape of at least one of a front surface and a back surface of the ophthalmic lens to reduce the aberration resulting from the modeled optical system.

**2.** The method of designing the aspheric ophthalmic lens according to claim 1, wherein the step of calculating the aberration resulting from the modeled optical system uses a Zernike polynomial as a calculation expression.

**3.** The method of designing the aspheric ophthalmic lens according to claim 1 or 2, wherein the designing of at least one of the front surface and the back surface of the ophthalmic lens uses the following mathematic expression (i);

$$z = \frac{(1/R)\,h^2}{1 + \sqrt{1 - (\frac{1}{R})^2(Q+1)\,h^2}} + A\,h^4 + B\,h^6 + C\,h^8 + D\,h^{10} \qquad \cdots (1)$$

**4.** The method of designing the aspheric ophthalmic lens according to any of claims 1 to 3, wherein the predetermined angle falls within a range of 1° to 10° in a horizontal direction with respect to the optical axis of the eye.

**5.** The method of designing the aspheric ophthalmic lens according to claim 4, wherein the predetermined angle additionally falls within a range of 3° or less in a vertical direction with respect to the optical axis of the eye.

**6.** The method of designing the aspheric ophthalmic lens according to any of claims 1 to 5, wherein the ophthalmic lens is an intraocular lens.

# FIG. 1

Ear Side

1

Optical Axis

Rotation Point

$\theta$

Visual Axis

Nose Side

EUROPEAN SEARCH REPORT

Application Number

EP 09 15 6602

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2007/128423 A (ZEISS CARL MEDITEC AG [DE]; LESAGE CEDRIC [FR]; GERLACH MARIO [DE]) 15 November 2007 (2007-11-15)<br>* page 3, line 37 - page 4, line 19 *<br>* page 7, line 35 - page 11, last line ; figures 1-13 *<br>& DE 10 2006 021521 A1 (ZEISS CARL MEDITEC AG [DE]) 8 November 2007 (2007-11-08) | 1-6 | INV.<br>G02C7/04<br>A61F2/16 |
| X | LIOU L H ET AL: "Anatomically accurate, finite model eye for optical modeling" JOURNAL OF THE OPTICAL SOCIETY OF AMERICA A, OPTICAL SOCIETY OF AMERICA, US, vol. 14, no. 8, 1 August 1997 (1997-08-01), pages 1684-1695, XP008082210 ISSN: 1084-7529<br>* the whole document *<br>* page 1688, right-hand column, line 16 - line 34; figure 4 * | 1-6 | |
| X | EP 1 329 206 A (EURO LENS TECHNOLOGY S P A [IT]) 23 July 2003 (2003-07-23)<br>* paragraph [0008] - paragraph [0021]; figures 1-7 * | 1-6 | TECHNICAL FIELDS SEARCHED (IPC)<br>G02C<br>A61F |
| X | EP 1 857 077 A (ALCON MFG LTD [US] ALCON RES LTD [US]) 21 November 2007 (2007-11-21)<br>* paragraph [0004] - paragraph [0019] *<br>* paragraph [0043] - paragraph [0080]; figures 1-16B * | 1-6 | |

The present search report has been drawn up for all claims

| Place of search | Date of completion of the search | Examiner |
|---|---|---|
| Munich | 15 June 2009 | Bratfisch, Knut |

**ANNEX TO THE EUROPEAN SEARCH REPORT
ON EUROPEAN PATENT APPLICATION NO.**                     EP 09 15 6602

This annex lists the patent family members relating to the patent documents cited in the above-mentioned European search report.
The members are as contained in the European Patent Office EDP file on
The European Patent Office is in no way liable for these particulars which are merely given for the purpose of information.

15-06-2009

| Patent document cited in search report | Publication date | Patent family member(s) | Publication date |
|---|---|---|---|
| WO 2007128423 A | 15-11-2007 | AU 2007247491 A1<br>CN 101437468 A<br>DE 102006021521 A1<br>EP 2034928 A1<br>KR 20090020588 A<br>US 2009125105 A1 | 15-11-2007<br>20-05-2009<br>08-11-2007<br>18-03-2009<br>26-02-2009<br>14-05-2009 |
| DE 102006021521 A1 | 08-11-2007 | AU 2007247491 A1<br>CN 101437468 A<br>EP 2034928 A1<br>WO 2007128423 A1<br>KR 20090020588 A<br>US 2009125105 A1 | 15-11-2007<br>20-05-2009<br>18-03-2009<br>15-11-2007<br>26-02-2009<br>14-05-2009 |
| EP 1329206 A | 23-07-2003 | AT 332511 T<br>IT MI20012765 A1 | 15-07-2006<br>23-06-2003 |
| EP 1857077 A | 21-11-2007 | AR 060959 A1<br>AT 429874 T<br>AU 2007202215 A1<br>BR PI0705740 A<br>CA 2588487 A1<br>CN 101199437 A<br>JP 2007313313 A<br>KR 20070111399 A<br>US 2007268453 A1 | 23-07-2008<br>15-05-2009<br>06-12-2007<br>10-06-2008<br>17-11-2007<br>18-06-2008<br>06-12-2007<br>21-11-2007<br>22-11-2007 |

EPO FORM P0459

For more details about this annex : see Official Journal of the European Patent Office, No. 12/82